(19) Europäisches Patentamt
European Patent Office
Office européen des brevets

(11) Publication number: 0 265 127 A1

# (12) EUROPEAN PATENT APPLICATION

(21) Application number: 87308924.7

(22) Date of filing: 08.10.87

(51) Int. Cl.⁴: G01N 33/53 , C07K 7/62 , C12Q 1/04

(30) Priority: 09.10.86 GB 8624253

(43) Date of publication of application: 27.04.88 Bulletin 88/17

(84) Designated Contracting States: BE CH DE ES FR GB IT LI NL

(71) Applicant: Harvey, Wilson
53 Punchcroft New Ash Green
Dartford Kent DA3 8HR(GB)

Applicant: Wilson, Michael
34A De Beauvoir Road
London N1 5SU(GB)

(72) Inventor: Harvey, Wilson
53 Punchcroft New Ash Green
Dartford Kent DA3 8HR(GB)
Inventor: Wilson, Michael
34A De Beauvoir Road
London N1 5SU(GB)

(74) Representative: Percy, Richard Keith
Patent Department National Research
Development Corporation 101 Newington
Causeway
London SE1 6BU(GB)

## (54) Endotoxin assay.

(57) It has been a problem that the known LAL method of endotoxin assay suffers from batch-to-batch differences in sensitivity, does not measure absolute amounts of endotoxin and gives some false positives. The assay of the invention quantitates endotoxin by making use of the binding interaction of the lipopolysaccharide (LPS) component of endotoxin with a polymyxin or octapeptin or a similar cyclic peptide. The cyclic peptide or the LPS is labelled, preferably enzyme-labelled.

EP 0 265 127 A1

Xerox Copy Centre

# ENDOTOXIN ASSAY

## Background of the Invention

### 1. Field of the Invention

This invention relates to a quantitative assay for endotoxins and lipopolysaccaride components thereof. Endotoxins are the fever-producing "pyrogens" associated with infections by Gramnegative bacteria. In such infections they can give rise to the fatal condition known as "endotoxic shock". They are heat-stable complexes formed of lipopolysaccharide (LPS), which is the active component, and lipid A-associated proteins.

### 2. Description of the prior art

The potent biological activity of LPS, its ubiquitous occurrence and its ability to withstand sterilisation have caused considerable problems in the pharmaceutical industry where all intraveneous preparations must be marketed as being "pyrogen-free". This industry is therefore in need of an assay for the detection and quantitation of endotoxin or LPS in starting materials used in the production of parenteral drugs, for in-process monitoring of endotoxin or LPS levels, and is a "final release test". While the need for an endotoxin assay in the pharmaceutical industry and for clinical use as well established, there is increasing interest in the use of such an assay in the public health sector for screening public water supplies for faecal organisms and in monitoring institutional supplies for the presence of high Gram-negative counts necessary for the growth of Legionella species.

At present, the only widely used methods of screening for endotoxins involve animal testing or the use of the Limulus Amoebocyte Lysate (LAL) assay. The LAL assay employs lysates of haemolymph amoebocytes from the horseshoe crab (Limulus polyphemus) which coagulate in the presence of endotoxins. The LPS component reacts with the LAL to trigger an enzyme cascade which results in formation of a network of proteins causing the coagulation. The coagulate takes the form of a "clot" which can be seen with the naked eye or in a turbidity which can be measured spectrophotometrically.

Although it is capable of great sensitivity, the LAL assay suffers from many disadvantages:

(a) The sensitivity of the test varies greatly depending on the specific properties of the particular lysate preparation used (i.e. cells from different crabs exhibit differences in sensitivity to LPS). There is, therefore, tremendous variation in sensitivity from batch-to-batch.

(b) Endotoxins of different bacteria vary with respect to their ability of initiate gelation of the lysate. Quantitation assays using LAL are therefore subject to considerable errors since they generally utilise a standard E. coli endotoxin for calibration and they therefore only measure the relative biological efffectiveness of the LPS - not the absolute amount of LPS present.

(c) False positive reactions are common since gelation of LAL can be initiated by a range of macromolecules - thrombin, thromboplastin, RNA, RNase, trypsin and trypsin-like enzymes, lipoteichoic acid and peptidoglycan fragments.

(d) False negative reactions can be caused by trypsin inhibitors, EDTA and other calcium binding reagents, high salt concentrations and semi-synthetic penicillins.

(e) Testing human blood for the presence of endotoxin is complicated by the need to remove inhibitors of endotoxin present in blood plasma before the LAL assay is carried out. US Patent 3,994,391 proposes a complicated procedure involving adsorption of the endotoxin on a polymer to avoid this problem.

(f) It is expensive.

Y.D. Karkhanis et al ., Analytical Biochemistry 85, 595-610 (1978), have described an assay for endotoxin which involves treatment of the LPS with sulphuric acid to release 2-keto-3-deoxyoctonate (KDO), followed by reactions with periodic acid ($HIO_4$), sodium arsenite ($NaAsO_2$) and thiobarbituric acid, which produce a red colour. Important disadvantages of this method are that in some endotoxins the LPS does not contain KDO and that the sensitivity limit of about 0.1 mg endotoxin is well below that of the LAL assay.

It has been known for a long time that the cyclic peptide antibiotic polymyxin B binds to enterobacterial LPS, see E. Neter et al., J. Immunology 80, 66-72 (1958), especially at page 70, right-hand column, lines 4 to 15 from bottom.

D.C. Morrison and D.M. Jacobs, Immunochemistry 13 , 813-818 (1976) reported that polymyxin B interacts stoichiometrically with LPS to form a stable molecular complex, probably in a 1:1 ratio.

The binding of polymyxin B to LPS was found in the mid-1970s to take away many of th biological activities of LPS, as reviewed by D.C. Morrison and B.J. Curry, J. Immunological Methods 27, 83-92 (1979) at page 84, middle. In the same paper Morrison and Curry reported that LPS preparations from Gram-negative bacteria stimulated the spleen cells of mice and that the addition of polymyxin B had no effect on the mitogenic response of the spleen cells. It was concluded that the use of polymyxin B was an inappropriate test to detect endotoxin contamination in biological samples.

A. Issekutz, J. Immunological Methods 61, 275-281 (1983) used polymyxin B bound to cyanogen bromide-activated Sepharose 4B to remove naturally-occurring as well as purified LPS from various solutions, demonstrating that lipid A-associated proteins do not interfere with the binding of polymyxin B to endotoxins.

Polymyxin B is known to combine with LPS from a wide range of bacteria, see J. Wiegel and L. Quandt, J. Gen. Microbiol. 128, 2261-2270 (1982).

H.S. Warren et al., Antimicrobial Agents and Chemotherapy 28, 107-112 (1985) have shown that other polymyxins, viz. colistin and colistin nonapeptide, bind to LPS and have anti-endotoxin activity.

## Summary of the Invention

Contrary to the indications of Morrison and Curry, supra, it has now been found that the binding of polymyxin B and similar cyclic peptides to endotoxin can be used in an assay for endotoxin, or the LPS component thereof, and that such an assay can be used quatitatively. What is novel and inventive herein is the discovery that the polymyxin B - LPS binding effect is detectable and quantitatable at the low concentrations of LPS present in analytes and that the prejudice against an assay based on this interaction is not well founded. That this is a significant achievement is substantiated by the fact that no such assay has been previously disclosed, whether despite or because of the prior pieces of knowledge referred to above, and notwithstanding the rapid commercialisation of assay kits made possible during the last 9 years or so by enzyme labelling.

The present invention provides a method of quantitative assay of endotoxin or a LPS component thereof in an analyte, which comprises incubating the analyte with a cyclic peptide of formula (1):

```
                                 L-DAB ──→ A6 ──→ A7
                                ↗
X ──(4-amino)──→ L-DAB                         ↓        (1)
                                ↖
                                 A10 ←── L-DAB ←── L-DAB
```

wherein:

the arrowed direction of bonding is from amino to carboxyl; divalent DAB represents the residue of 2,4-diaminobutyric acid bonded through its 2-amino and 1-carboxyl groups; of formula

```
──→ NH-CH ── CO ──→
      |                       (2)
     CH2 ── CH2-NH2
```

the trivalent DAB represents the residue of 2,4-diaminobutyric acid bonded through its 4-amino, 2-amino and 1-carboxyl group, the X-substituent being bonded through the 4-amino group and the ring members through the 2-amino and 1-carboxyl group, of formula :

```
(from ring) ──→ NH-CH ── CO ──→ (to ring)
                  |                          (3)
                 CH2 ── CO2-NH ←── (from X);
```

X represents R→ D or L -DAB → ($A^{2/3}$) → (4)
where R represents a hydrogen atom or an acyl group and $A^{2/3}$represents a dipeptide or single amino acid residue or is omitted; $A^6$ represents a neutral amino acid, [especially D -Leu or D -Phe] residue;
$A^7$ represents a neutral amino acid, [especially L -Thr, L -Ile or L -Phe] residue; and
$A^{10}$ represents a neutral amino acid, [especially L -Leu or L -Thr] residue;
and derivatives thereof by substitution of pendant 4-amino groups of divalent DAB groups, whereby the LPS present in the analyte binds to the cyclic peptide and determining the amount of endotoxin LPS which becomes bound to the cyclic peptide. Either the LPS or the cyclic peptide can be labelled for this purpose.

The invention also provides a diagnostic kit for use in the above-defined method comprising, in separate containers (a) labelled lipopolysaccharide and (b) immobilised cyclic peptide of formula (1) or (a) labelled cyclic peptide of formula (1) and (b) as a standard, lipopolysaccharide.

## Description of the preferred embodiments

The literature evidence to date indicates that the diaminobutyric ring amino acids are primarily responsible for the binding to LPS. The pendant amino groups are readily protonated to give catonic groups which appear to bind strongly to receptor sites on the LPS. Other amino acids with pendant amino groups such as lysine or arginine could be substituted for some or all of the diaminobutyric acid and the number and order of such groups in the ring could be varied. Such modifications are likely to give rise to similar LPS-binding effects.

The nature of the side-chain X does not appear to be critical. In particular, the acyl group R can be dispensed with, as in the nonapeptides referred to above in which R is hydrogen. The R group could be modified in other ways to give similar LPS-binding effects. $A^{2/3}$ is preferably D or L -DAB or includes such a residue within a dipeptide.

Within the cyclic peptide ring, $A^6$, $A^7$ and $A^{10}$ are particularly variable and can be a residue of any neutral amino acid such as glycine, alanine, valine, leucine, isoleucine, phenylalanine, tyrosine, tryptophan, serine, threonine, methionine, cysteine or proline. $A^6$ is conveniently a residue of D -leucine or phenylalanine, $A^7$ a residue of L -threonine, leucine, isoleucine or phenylalanine and $A^{10}$ a residue of any of the amino acids listed for $A^7$, especially L -leucine or threonine.

The preferred general classes of cyclic peptides within formula (1) are the polymyxins and the octapeptins. Polymyxins have a side-chain (X) group of formula (5):
R → L -DAB → L -Thr → D or L -DAB → (5)
and $A^{10}$ is L -Thr, while octapeptins have a side-chain (X) group of formula (6):
R → L -DAB → (6)
and $A^{10}$ is L -Leu. In either class the terminal acyl (R) group can be replaced by hydrogen. Typical acyl groups are straight or branched chain alkanoyl groups, especially those having from 7 to 10 carbon atoms, which can be substituted (by groups other than alkyl), for example by hydroxyl, carboxyl or sulphonyl groups.

Table 1 below shows some known polymyxins and octapeptins for use in the present invention.

TABLE 1

Structures of polymyxins and octapeptins (adapted from Storm, Rosenthal and Swanson, Ann. Rev. Biochem. 46, 723-764 (1977) at page 726)

Octapeptin Acyl → D-DAB → L-DAB → L-DAB → $A^6$ → $A^7$ → L-DAB → L-DAB → L-Leu

Polymyxin Acyl → L-DAB (1) → L-Thr (2) → $A^3$ (3) → L-DAB (4) → L-DAB (5) → $A^6$ (6) → $A^7$ (7) → L-DAB (8) → (9) L-DAB (10) → L-Thr

| Compound | Acyl(R) | $A^3$ | $A^6$ | $A^7$ |
|---|---|---|---|---|
| Polymyxin A | MOA | D-DAB | D-Leu | L-Thr |
| Polymyxin $B_1$ | MOA | L-DAB | D-Phe | L-Leu |
| $B_2$ | MOA | L-DAB | D-Phe | L-Leu |
| Polymyxin $D_1$ | MOA | D-Ser | D-Leu | L-Thr |
| Polymxin $E_1$ (colistin A) | MOA | L-DAB | D-Leu | L-Leu |
| $E_1$ (colistin B) | IOA | L-DAB | D-Leu | L-Ile |
| Circulin A | MOA | L-DAB | D-Leu | L-Leu |
| Octapeptin $A_1$ | Beta-OH MD | - | D-Leu | L-Ile |
| $A_2$ | Beta-OH MN | - | D-Leu | L-Leu |
| $A_3$ | Beta-OH ND | - | D-Leu | L-Leu |

TABLE 1 (continued)

Structures of polymyxins and octapeptins (adapted from Storm, Rosenthal and Swanson, Ann. Rev. Biochem. 46, 723-764 (1977) at page 726)

Octapeptin Acyl → D-DAB ——→ L-DAB → L-DAB → $A^6$ → $A^7$ → L-DAB → L-DAB → L-Leu (L-Leu joined back to the first L-DAB, forming a ring)

Polymyxin Acyl → L-DAB (1) → L-Thr (2) → $A^3$ (3) → L-DAB (4) → L-DAB (5) → $A^6$ (6) → $A^7$ (7) → L-DAB (8) → (9) L-DAB (10) → L-Thr (L-Thr joined back to L-DAB 4, forming a ring)

| Compound | Acyl(R) | $A^3$ | $A^6$ | $A^7$ |
|---|---|---|---|---|
| Octapeptin $B_1$ | Beta-OH MD | - | D-Leu | L-Phe |
| $B_2$ | Beta-OH MN | - | D-Leu | L-Phe |
| $B_3$ | Beta-OH ND | - | D-Leu | L-Phe |
| Octapeptin $C_1$ | Beta-OH MOA | - | D-Phe | L-Leu |

MOA = 6-methyloctanoic acid; IOA = 6-methylheptanoic acid; MD = 8-methyldecanoic acid;
MN = 8-methylnonanoic acid; ND = n-decanoic acid.

Polymyxins contain 10 amino acids. Their "nonapeptides" (not shown in Table 1) have a hydrogen atom in place of the acyl (R) group.

The octapeptins differ from the polymyxins in that they contain only 8 amino acids, as the name implies, and have a long β-hydroxy fatty acid residue linked to the peptide. Typical amino acids found in the octapeptins are leucine, phenylalanine, and DAB. The octapeptin fatty acids include the straight-chain 3-hydroxydecanoic acid and the branched-chain 3-hydroxy-3-methyl-nonanoic or 3-hydroxy-8-methyldecanoic acids. Octapeptin classes A and B each contain a mixture of these fatty acids.

The cyclic peptides of formula (1) include those in which pendant amino groups, notably the pendant 4-amino groups of DAB residues, are substituted. It is known that sodium suphomethyl derivatives of a polymyxins behave similarly to polymyxins in their antibacterial properties, believed also to be brought about primarily by the ring structure of the peptide, see M. Barnett et al., British Journal of Pharmacology 23, 552-574 (1964). Thus, the amino groups can be substituted, for example by alkyl, sulphoalkyl (-alkylene-$SO_3M$) or carboxyalkyl (-alkylene-COOM), M being hydrogen or an alkali metal atom, for example sodium.

The most preferred cyclic peptides for use in the invention are polymyxin B, which is a mixture of polymyxin $B_1$ and $B_2$, polymyxin $B_1$ and $B_2$, polymyxin E or a nonapeptide of any of these.

Polymyxin B is commercially available. Its components $B_1$ and $B_2$ can be obtained by chromatographic separation. Other compounds in which R is acyl, especially alkanoyl, can be obtained by acylating the nonapeptide after protecting all other primary amino groups and then de-protecting them after the acylation. Alkanoyl halides are well known for this purpose. Alkanoyl groups having at least 6, preferably 7 to 11, carbon atoms and which are optionally branched and/or substituted by groups other than alkyl, are preferred. Protection and de-protection methods well known in peptide systhesis can be used. Other cyclic peptides of formula (1) are commercially available or can be prepared by analogous procedures.

The assay can be carried out in any manner in which the amount of polymyxin B - LPS binding is determined by applying a label to one of the binding partners and measuring the amount of it found in the other partner. Since it is not normally convenient to either immobilise the analyte or to label it, preferred heterogeneous assay methods are those involving displacement (quasi-competition) or competition. Thus in one form of the assay the analyte which contains LPS and a standard, labelled, LPS preparation compete for a limited amount of immobilised polymyxin B, and the amount of label bound to the polymyxin B is then quantitated. Another form of heterogeneous assay is the direct assay in which the analyte LPS is immobilised and the amount of labelled polymyxin B bound to the LPS is measured.

Analytes to which the assay of the invention can be applied include the following: media containing biological preparations, especially extracts from cells, drugs, body fluids, and solids and liquids suspected of being bacterially contaminated. The invention is of particular interest in the fields of environmental health and clininal microbiology, for the detection of contaminated food and drink and for bacterial infection of urine of a patient. Infections by Gram-negative bacteria e.g. Salmonella, Escherichia and Proteus, can be detected using an endotoxin assay, since all such bacteria produce endotoxin. A quantitative assay according to the invention is used for this purpose. While about 95% of urinary tract infections are due to Gram-negative bacteria, the Gram-positive bacteria can be detected by combining the endotoxin assay with an appropriate additional assay for Gram-positive bacteria, for example a test based on the hydrolysis of L-pyrrolidonyl-beta-napthylamide as described by T.R. Oberhofer, Diagn. Microbiol. Infect. Dis. 4 43-47 (1986).

Conveniently the labels are enzyme labels, whereby ELISAs can be used. Radiolabels and fluorescent labels are alternatives.

The sensitivity of the assay of the invention can be increased by using the Colin Self cycling amplification method, see his European Patent Applications 27036A and 58539A and the reference thereto given herein in Example 2. The sensitivity of detection can be enhanced by using horseradish peroxidase as the enzyme label and an enhanced luminescent reaction for detection of the peroxidase, see e.g. US Patent 4,598,044 (NRDC).

The following Examples illustate the invention.

## EXAMPLE 1

This Example illustrates a method of assay in which analyte containing endotoxin LPS is incubated with insolubilised polymyxin B and the amount of LPS bound is determined indirectly. The basis of this determination is first to measure the binding capaicity of the polymyxin B for LPS. After the test solution containing LPS has been incubated with the polymyxin B, isotopically-labelled LPS is then added and,

following incubation and centrifugation, the amount of bound labelled LPS is determined. The amount of bound LPS can be most conveniently determined by a subtractive method, in which the amount of labelled LPS found in the supernatant is subtracted from the total amount of labelled LPS used. Knowing the binding capacity of the immobilised polymyxin, the amount of LPS in the test solution can readily be calculated from:

quantity of LPS in test solution = binding capacity - quantity of bound labelled LPS.

Polymyxin B was coupled to cyanogen bromide-activated Sepharose 4B as follows. 1.6 g of cyanogen bromide-activated Sepharose 4B (Pharmacia) was swollen and washed with 1 mM HCl, resuspended in 5 ml of -0.1 M $NaHCO_3$ buffer, pH 8.3, containing 0.5 M NaCl and centrifuged at low speed (100 rpm 3-5 minutes) to sediment the gel and the excess buffer was discarded. This was replaced by 2.5 ml of the same buffer in which 50 mg of polymyxin B sulphate (Sigma) had been disolved. The tube containing the Sepharose was then sealed and slowly rotated overnight (16-18 hours) at 4°C. Following this incubation, the Sepharose was washed twice with 10-15 ml of 0.1 M $NaHCO_3$, 0.5 M NaCl, pH 8.3, buffer. Excess buffer was discarded and replaced by 10 ml of 1 M ethanolamine, pH 9, in order to block the remaining reactive groups. After 2 hours of incubation at room temperature, the Sepharose was allowed to sediment and the ethanolamine solution was removed and the tube filled with 0.1 M sodium acetate buffer, pH 4.0, containing 0.5 M NaCl.

$^{14}C$-Lipopolysaccharide was prepared as follows. Escherichia coli (NCTC 11560) was cultivated in 2.5 litres of Brain Heart Infusion broth (Oxoid Ltd.) containing 50 µCi of $^{14}C$-acetate (Amersham International). After 24 hours incubation at 37°C the cells were harvested by centrifugation, washed in saline and lyophilised. The lyophilised cells were extracted 3 times with phenol/water at 68°C, by the method of Westphal and Luderitz, Angew. Chem. 66, 407-417 (1953), and the aqueous phases pooled, dialysed and lyophilised. The crude lipopolysaccharide (LPS) was purified by treatment with DNase, RNase, and pronase and was then ultracentrifuged twice at 100,000 g for 1 hour.

To show that LPS can block the binding of $^{14}C$-LPS to polymyxin B/Sepharose 4B (PB/S4B) the following experiment was performed. 0.4 ml of a 1:1 suspension of PB/S4B in water was added to solutions containing various concentrations of standard E. coli LPS preparation (0-200 µg/ml) and these were shaken at room temperature for 30 minutes. 50 µg of $^{14}C$-LPS (specific activity 21.3 dmp/µg) was added to each solution and these were shaken for another 30 minutes. Each suspension was centrifuged, the supernatant removed, the gel washed with 0.5 ml water, centrifuged and the pooled supernatants from each gel counted in an LKB liquid scintillation counter.

To determine the binding capacity of the PB/S4B various quantities of $^{14}C$-LPS were added to 0.4 ml of the PB/S4B gel suspension which were shaken at room temperature for 30 minutes. Supernatants and washings were pooled and counted as described above. Results are shown in the Table 2 below from which the binding capacity was found to be 100 µg LPS per 0.4 ml of gel.

TABLE 2

| $^{14}C$-LPS added (µg) | Bound $^{14}C$-LPS (dpm) |
|---|---|
| 10 | 189 |
| 20 | 417 |
| 30 | 595 |
| 40 | 771 |
| 50 | 1066 |
| 60 | 1296 |
| 70 | 1484 |
| 80 | 1717 |
| 90 | 1880 |
| 100 | 2074 |
| 110 | 2359 |
| 120 | 2355 |
| 130 | 2454 |

As a test of the assay 50 µg of Salmonella abortus-equi LPS was shaken for 30 minutes with 0.4 ml of PB/S4B followed by addition of 100 µg $^{14}C$-LPS and shaken for a further 30 minutes. The supernatant and washing was counted as described above. Results were as follows:
100 µg $^{14}C$-LPS : 2107.2 dpm
supernatants : 707.3 dpm
therefore bound $^{14}C$-LPS = 1399.9 dpm = 65.7 µg
therefore LPS content of "unknown" = 110-65.7 = 44.3 µg.

The assay was quick and easy to perform and enabled a reasonably accurate estimation of the LPS content of an "unknown" solution.

The sensitivity of the assay is limited by the specific activity of the labelled LPS employed. The $^{14}C$-LPS was prepared in the laboratory and, although this had only a low specific activity (21.3 dpm/µg), it enabled the detection of µg quantities of LPS. The specific activity of the labelled LPS could very easily be increased by specialist laboratories, enabling the detection of much lower concentrations of LPS.

EXAMPLE 2

This Example illustrates a displacement, i.e. quasi-competitive, ELISA in which the analyte endotoxin LPS and a known amount of immbilised LPS compete for a limited amount of enzyme-conjugated polymyxin B.

Materials

96-well flat-bottomed microassay plates (commercially available) Pure LPS from Samonella abortus-equi (commercially available) Polymyxin B-alkaline phosphatase conjugate prepared by glutaraldehyde coupling using the method of Avrameas and Ternynck, Immunochem. 8, 1175-1179 (1971)
p-nitrophenyl phosphate (commercially available)
alcohol dehydrogenase (commercially available)
diaphorase (commercially available)
NADP (commercially available)
Multi-channel spectrophotometer (e.g. Titertek Multiskan)

Method

1. Test samples are incubated with a molar excess of polymyxin B-alkaline phosphatase in polystyrene tubes.

2. The mixtures are added to 96-well polypropylene or PVC microassay plates coated with a standard LPS preparation. Further incubation allows the excess polymyxin B-alkaline phosphatase to bind to the immobilised LPS on the plastic surface. Unbound material is removed by rinsing.

3. Enzyme activity may be detected by:

(a) incubation with an appropriate phosphate substrate (e.g. p-nitrophenyl phosphate) to yield a coloured reaction product which is measured on a multi-channel spectrophotometer or assessed visually by comparison with a coloured chart or appropriate reference standards in the assay plate, or

(b) an "amplification" method where the initial substrate is NADP and the secondary amplifying enzyme system is alcohol dehydrogenase and diaphorase, yielding a coloured reaction product which monitored at 490 nm, C.H. Self, J. Immunol. Methods 76, 389-393 (1985).

In both cases the absorbance (colour) is inversely proportional to the concentration of endotoxin in the sample.

EXAMPLE 3

This Example illustrates a displacement ELISA of the reverse sense to that of Example 2, in which the analyte endotoxin LPS and labelled LPS (enzyme conjugate) compete for a limited amount of immobilised polymyxin B.

Materials

As for Example 2 except that the LPS-alkaline phosphatase conjugate is prepared by cyanogen bromide coupling, see Axen et al., Nature 214, 1302 (1967).

Method

1. Polystyrene microtitre plates are coated with a limited amount of polymyxin B by direct adsorption and rinsed.

2. The text sample (or a dilution in PBS) is added and incubated to allow binding. Unbound material is removed by rinsing.

3. The LPS-alkaline phosphatase conjugate is added and, following incubation, excess conjugate is removed by rinsing.

4. Enzyme activity is quantitated spectrophotometrically as described for Example 2. Absorbance is inversely proportional to the concentration of endotoxin in the sample.

EXAMPLE 4

This Example illustrates a direct ELISA in which the analyte endotoxin LPS is immobilised and the amount of labelled polymyxin B bound to it is measured.

Materials

As for Example 2.

Method

1. Test samples are incubated in 96-well polypropylene or PVC microassay plates; pure LPS standards are added to other wells.
2. All wells are rinsed with saline.
3. Polymyxin B-alkaline phosphatase conjugate is added to all wells, incubated, and rinsed.
4. Enzyme activity is detected as in Example 2.

## Claims

1. A method of quantitative assay of endotoxin or a lipopolysaccharide component thereof in an analyte, which comprises incubating the analyte with a cyclic peptide of formula (1):

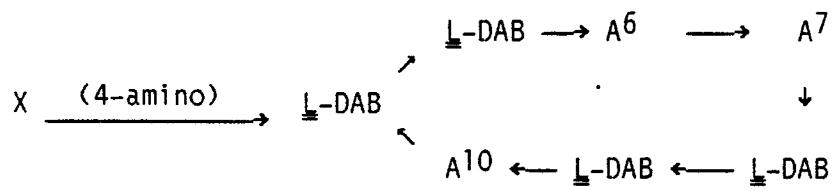

(1)

wherein:
the arrowed direction of bonding is from amino to carboxyl; divalent DAB represents the residue of 2,4-diaminobutyric acid bonded through its 2-amino and 1-carboxyl groups of formula

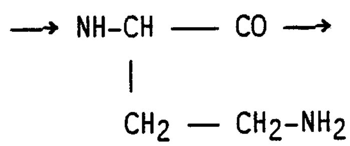

(2)

the trivalent DAB represents the residue of 2,4-diaminobutyric acid bonded through its 4-amino, 2-amino and 1-carboxyl groups, the X-substituent being bonded through the 4-amino group and the ring members through the 2-amino and 1-carboxyl group, of formula:

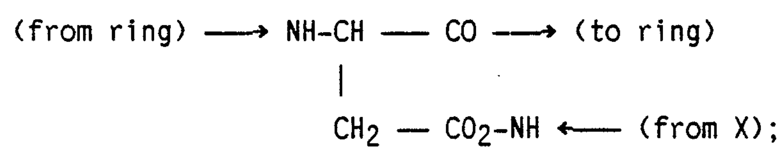

(3)

X represents R → D or L -DAB → ($A^{2/3}$)→ (4)
where R represents a hydrogen atom or an acyl group and $A^{2/3}$represents a dipeptide or single amino acid residue or is omitted;
$A^6$ represents a residue of a neutral amino acid;
$A^7$ represents a residue of a neutral amino acid; and
$A^{10}$ represents a residue of a neutral amino acid;
and derivatives thereof by substitution of pendant 4-amino groups of divalent DAB groups, and determining the amount of endotoxin lipopolysaccharide which becomes bound to the cyclic peptide.

2. A method according to claim 1 wherein $A^6$ is D -Leu or D -Phe, $A^7$ is L -Thr, L -Ile or L -Phe and $A^{10}$ is L -Leu or L -Thr.

3. A method according to Claim 1 or 2 wherein the cyclic peptide is a polymyxin having a side-chain (X) group of formula (5)
R → L -DAB → L -Thr → D or L-DAB → (5)
and in which $A^{10}$ is a L -threonine residue.

4. A method according to Claim 3 wherein the cyclic peptide is polymyxin B or E or a nonapeptide thereof, that is to say a cyclic peptide of formula (1) wherein :-
$A^6$ represents D -Phe or D -Leu;

$A^7$ represents L-Leu or when $A^6$ represents D-Leu, L-Ile;
$A^{10}$ represents L-Thr; and
X represents R → L-DAB → ($A^{2/3}$) → , where
$A^{2/3}$ represents L-Thr → L-DAB; and
R represents a hydrogen atom or 6-methyloctanoyl or, when $A^7$ represents L-Ile, 6-methylheptanoyl.

5. A method according to Claim 1, 2, 3, or 4 wherein the assay is a direct, displacement or competitive ELISA.

6. A method according to Claim 1, 2, 3, 4 or 5 wherein the analyte is a bacterially contaminated body fluid, food or beverage and the assay according to the said method is supplemented by an additional assay for Gram-positive bacteria.

7. A diagnostic kit for use in the method of Claim 1 comprising in separate containers (a) labelled lipopolysaccharide and (b) immobilised cyclic peptide of formula (1)

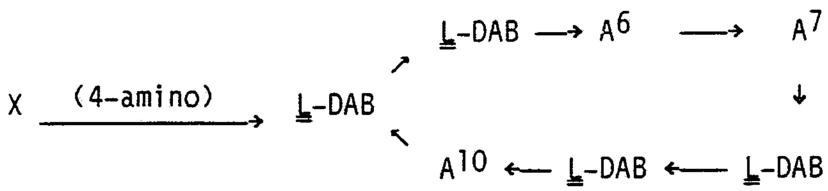
(1)

wherein:
the arrowed direction of bonding is from amino to carboxyl; divalent DAB represents the residue of 2,4-diaminobutyric acid bonded through its 2-amino and 1-carboxyl groups of formula

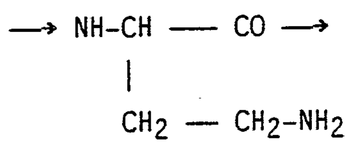
(2)

the trivalent DAB represents the residue of 2,4-diaminobutyric acid bonded through its 4-amino, 2-amino and 1-carboxyl groups, the X-substituent being bonded through the 4-amino group and the ring members through the 2-amino and 1-carboxyl group, of formula:

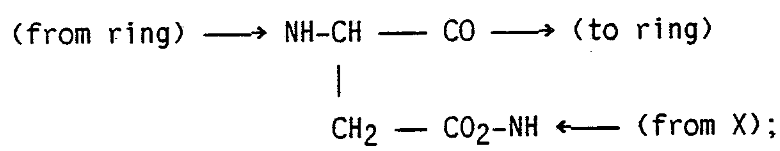
(3)

X represents R → D or L-DAB → ($A^{2/3}$) → (4)
where R represents a hydrogen atom or an acyl group and $A^{2/3}$represents a dipeptide or single amino acid residue or is omitted;
$A^6$ represents a residue of a neutral amino acid;
$A^7$ represents a residue of a neutral amino acid; and
$A^{10}$ represents a residue of a neutral amino acid;
and derivatives thereof by substitution of pendant 4-amino groups of divalent DAB groups, or (a) labelled cyclic peptide of formula (1) and (b) as a standard, endotoxin lipopolysaccharide.

8. A diagnostic kit according to Claim 7 wherein $A^6$ is D-Leu or D-Phe, $A^7$ is L-Thr, L-Ile or L-Phe and $A^{10}$ is L-Leu or L-Thr.

9. A kit according to Claim 7 or 8 wherein the cyclic peptide is a polymyxin having a side-chain (X) group of formula (5):
R → L-DAB → L-Thr → D or L-DAB → (5)
and in which $A^{10}$ is a L-threonine residue.

10 A kit according to Claim 9 wherein the cyclic peptide is polymyxin B or E or a nonapeptide thereof, that is to say a cyclic peptide of formula (1) wherein:
$A^6$ represents D-Phe or D-Leu;
$A^7$ represents L-Leu or when $A^6$ represents D-Leu, L-Ile;
$A^{10}$ represents L-Thr; and

X represents R → L -DAB → ($A^{2/3}$) → , where
$A^{2/3}$ represents L -Thr → L -DAB; and
R represents a hydrogen atom or 6-methyloctanoyl or, when $A^7$ represents L -Ile, 6-methylheptanoyl.

European Patent Office

# EUROPEAN SEARCH REPORT

Application Number

EP 87 30 8924

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | JOURNAL OF IMMUNOLOGICAL METHODS, vol. 61, 1983, pages 275-281, Elsevier Biomedical Press, Elsevier Science Publishers B.V.; A.C. ISSEKUTZ: "Removal of gram-negative endotoxin from solutions by affinity chromatography" * Whole document * | 1-4,7-10 | G 01 N 33/53<br>C 07 K 7/62<br>C 12 Q 1/04 |
| A | CHEMICAL ABSTRACTS, vol. 102, no. 9, 4th March 1985, page 142, abstract no. 73634u, Columbus, Ohio, US; M. NIWA et al.: "Basic and applied studies of the Limulus test", & BACT. ENDOTOXIN 1984, 383-94 * Abstract * | 1-10 | |
| A,D | US-A-3 944 391 (N.S. HARRIS et al.) * Abstract; column 1, line 1 - column 3, line 20 * | 1-10 | |
| A,D | JOURNAL OF GENERAL MICROBIOLOGY, 1982, vol. 128, pages 2261-2270, SGM, GB; J. WIEGEL et al.: "Determination of the gram type using the reaction between polymyxin B and lipopolysaccharides of the outer cell wall of whole bacteria" * Abstract; page 2261, line 1 - page 2262, line 10; page 2265, line 44 - page 2267, line 27 * | 1-10 | TECHNICAL FIELDS SEARCHED (Int. Cl.4)<br>G 01 N<br>C 07 K<br>C 12 Q |

-/-

The present search report has been drawn up for all claims

| Place of search | Date of completion of the search | Examiner |
|---|---|---|
| THE HAGUE | 18-01-1988 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 87 30 8924

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | ANTIMICROBIAL AGENTS AND CHEMOTHERAPY, vol. 28, no. 1, July 1985, pages 107-112, American Society for Microbiology; H. S. WARREN et al.: "Binding and neutralization of bacterial lipopolysaccharide by colistin nonapeptide" * Abstract * --- | 1-10 | |
| A,D | ANN. REV. BIOCHEM., vol. 46, 1977, pages 723-763, Annual Reviews Inc.; D.R. STORM et al.: "Polymyxin and related peptide antibiotics" * Page 724-727 * ----- | 1-4,7-10 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 18-01-1988 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document